# FASCICULE DE BREVET EUROPEEN

(11) **EP 3 082 824 B1**
(45) Date de publication et mention de la délivrance du brevet: **13.06.2018**
(21) Numéro de dépôt: 14833231.5
(22) Date de dépôt: 18.12.2014
(51) Int. Cl.: A61K 31/55, A61K 9/00, A61K 9/08, A61K 47/12, A61P 3/00, A61P 3/02

(54) **MIRTAZAPINE INJECTABLE POUR LE TRAITEMENT DE LA PERTE D'APPÉTIT ET DES DÉSORDRES NUTRITIONNELS CHEZ LES CHATS**
INJIZIERBARES MIRTAZAPIN ZUR BEHANDLUNG VON APPETITVERLUST UND ERNÄHRUNGSSTÖRUNGEN BEI KATZEN
INJECTABLE MIRTAZAPINE FOR THE TREATMENT OF APPETITE LOSS AND NUTRITIONAL DISORDERS IN CATS

(30) Priorité: 20.12.2013 FR 1363226
(43) Date de publication de la demande: 26.10.2016
(73) Titulaire: VIRBAC, 06516 Carros (FR)
(72) Inventeur: DE MARI, Karine, F-06510 Carros (FR); SANQUER, Annaele, F-06100 Nice (FR)
(74) Mandataire: Gevers & Orès
(86) Numéro de dépôt international: PCT/IB2014/067079
(87) Numéro de publication internationale: WO 2015/092738

(56) Documents cités:
- WO-A1-2013/158130
- WO-A2-01/00196
- K.E. Avis, J. W. Levchuk: "Parenteral preparations"; "41" In: A. R. Gennaro: "Remington: the science and practice of pharmacy, 20th Edition", 2000, Lippincott, Baltimore, US, XP002728555, pages 780-786, page 781, colonne 1, alinéa 2-4 page 783, colonne 1, alinéa 3-4
- ROBIN M VOIGT ET AL: "Repeated mirtazapine nullifies the maintenance of previously established methamphetamine-induced conditioned place preference in rats", BEHAVIOURAL BRAIN RESEARCH, ELSEVIER, AMSTERDAM, NL, vol. 225, no. 1, 5 juillet 2011 (2011-07-05), pages 91-96, XP028284915, ISSN: 0166-4328, DOI: 10.1016/J.BBR.2011.07.009 [extrait le 2011-07-12]
- J.M. QUIMBY ET AL: "Mirtazapine as an appetite stimulant and anti-emetic in cats with chronic kidney disease: A masked placebo-controlled crossover clinical trial", THE VETERINARY JOURNAL, vol. 197, no. 3, 1 septembre 2013 (2013-09-01), pages 651-655, XP055134988, ISSN: 1090-0233, DOI: 10.1016/j.tvjl.2013.05.048 cité dans la demande
- MOHAMMAD-REZA ROUINI ET AL: "Pharmacokinetics of mirtazapine and its main metabolites after single intravenous and oral administrations in rats at two dose rates", DARU JOURNAL OF PHARMACEUTICAL SCIENCES, BIOMED CENTRAL LTD, LONDON, UK, vol. 22, no. 1, 7 janvier 2014 (2014-01-07), page 13, XP021173801, ISSN: 2008-2231, DOI: 10.1186/2008-2231-22-13
- QUIMBY J M ET AL: "The Pharmacokinetics of Mirtazapine in Cats with Chronic Kidney Disease and In Age-Matched Control Cats", JOURNAL OF VETERINARY INTERNAL MEDICINE, vol. 25, no. 5, September 2011 (2011-09), pages 985-989, ISSN: 0891-6640
- Anonymous: "Pharmacokinetics describes the relationship between drug concentration following administration.", , page 1, Retrieved from the Internet: URL:http://xenogesis.com/pharmacokinetics- pk/ [retrieved on 2016-10-19]

## Description

La présente invention se rapporte au domaine vétérinaire et a plus particulièrement pour objet la mise au point de médicaments pour le traitement de l'anorexie chez les animaux.

Le chat est un animal particulièrement sujet à l'anorexie, désordre qui peut se manifester dans plusieurs contextes ; elle peut par exemple résulter de modification brutale des aliments qui sont proposés, de situations stressantes pour l'animal, de changements environnementaux ou du vieillissement de l'animal. L'anorexie est également une manifestation clinique qui accompagne fréquemment les troubles de la santé du chat. Si l'élément déclencheur de l'anorexie nécessite d'être diagnostiqué et traité, il est également important d'aider rapidement l'animal à retrouver une alimentation normale pour éviter l'aggravation de sa pathologie et la survenue d'autres désordres. En outre, la présence d'une douleur quelle qu'en soit la cause (douleur dentaire, choc, troubles musculo-squelettiques aigus ou chroniques, etc...), de nausées et/ou de vomissements (suite à la prise de certains médicaments ou du mal des transports par exemple), d'une gêne physique partielle compliquant l'accès à la nourriture (présence d'une collerette, d'un plâtre ou d'un bandage), d'un trouble comportemental (tel que l'anxiété, la dépression, la démence, la phobie, l'aversion alimentaire) sont susceptibles de générer une anorexie. Enfin, l'anorexie est un désordre qui est fréquemment observé lors d'une hospitalisation et plus particulièrement lors de la période postopératoire ; là encore, il est important que l'animal retrouve rapidement une alimentation normale pour aider sa convalescence.

Le chat retrouve généralement son appétit en supprimant les causes de stress et lorsque son confort est amélioré ; mais cela n'est pas toujours suffisant, il est alors important de mettre en oeuvre un traitement médicamenteux qui stimule l'appétit et permet le retour à une alimentation normale.

La mirtazapine ((±)-1,2,3,4,10,14 b-hexahydro-2-méthylpyrazino-(2,1-a)-pyrido-(2,3-c)-benzazépine) est l'un des différents principes actifs utilisés pour le traitement de l'anorexie animale ; elle a la structure chimique suivante : 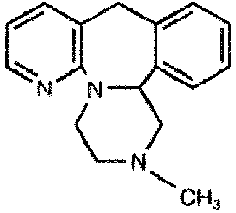 et est utilisée principalement comme antidépresseur en médecine humaine.

L'équipe de Quimby *et al.* a conduit plusieurs travaux sur la pharmacocinétique et la pharmacodynamique de la mirtazapine administrée par voie orale à une dose de 1,88 mg ou 3,75 mg chez le chat dont elle juge les effets satisfaisants (Quimby et al. J. vet. Pharmacol. Therap. 34, 388-396, 2010; J. Vet. Intern. Med. 2011;25;985-989 ; The Vet. Journal, 2013 ; WO 2013/158130) ; en outre, les auteurs expliquent leur réticence à administrer la mirtazapine par voie intra-veineuse car ils n'en attendent pas un effet intéressant, d'autant que cette voie n'avait jamais été explorée et risquait de provoquer des effets secondaires indésirables.

Il est cependant difficile d'administrer un médicament par la voie orale à un animal surtout si ce dernier refuse totalement de s'alimenter.

Dans ce contexte, la Demanderesse a mis en évidence que l'administration de mirtazapine par voie parentérale stimule efficacement l'appétit chez l'animal et présente des données pharmacocinétiques avantageuses par rapport à son administration orale.

La présente invention se rapporte ainsi à la mirtazapine (1,2,3,4,10,14b-hexahydro-2-méthylpyrazino(2,1-a)pyrido(2,3- c)benzazépine) pour son utilisation pour le traitement de la baisse ou de la perte d'appétit et/ou la prévention des désordres induits par une dénutrition chez un animal, caractérisée en ce que la mirtazapine est administrée par voie parentérale (sous-cutanée, intramusculaire, intradermique, intraveineuse) et formulée dans une composition vétérinaire injectable liquide comprenant une concentration de mirtazapine inférieure ou égale à 2% poids/volume. Dans le cas présent, l'injection de mirtazapine présente les avantages suivants :
- elle présente une bonne tolérance locale ;
- ainsi administrée, la mirtazapine produit un effet qui est moins dépendant de l'état de santé de l'animal et donc plus reproductible et plus fiable ;
- ce mode d'administration évite l'administration du médicament par gavage, méthode qui est particulièrement délicate chez le chat anorexique et/ou présentant un état nauséeux ;
- de façon inattendue, la pharmacocinétique de la mirtazapine est avantageuse car elle est plus lente et produit un effet prolongé ;
- la concentration plasmatique maximale (Cmax) est plus basse, ce qui évite les risques de surdosage et/ou d'intolérance ; ceci est particulièrement avantageux concernant la mirtazapine qui est connue pour avoir des effets toxiques sur le foie ;
- elle permet d'ajuster facilement la dose en fonction du poids du chat (dose ajustable en mg/kg) permettant ainsi de développer au mieux une dose à la fois efficace et une bonne tolérance systémique ;
- enfin, contrairement à une administration par voie orale, l'administration parentérale limite l'effet de premier passage hépatique réduisant ainsi les problèmes d'intolérance, de métabolisation ou de toxicité.

Cette utilisation de la mirtazapine vise les chats. Plus particulièrement, l'animal traité est un chat Abyssin, Abyssin à poil long, Américain à poil court, Américain à poil dur, American Bobtail, American Curl, American Shorthair, American Wirehair, Angora, Angora Turc, Asian, Australian Mist, Balinais, Balinais Traditionnel, Balinese, Bengal, Bengali, Birman, Bleu Russe, Bobtail Américain, Bobtail Japonais, Bobtail Russe, Bombay, British Longhair, British Shorthair, Burmese, Burmilla, California Rex, California Toyger, Californian Spangled, Celtic, Ceylan, Chantilly/Tiffany, Chartreux, Chat Caniche, Chat de Ceylan, Chat de Gouttière, Chat de l'Île de Man, Chat de Maison, Chat de Sibérie, Chat des Bois Norvégien, Chat des Forêts Norvégiennes, Chat du Sri-Lanka, Chat Nu, Chat Royal du Siam, Chat Turc de l'Ile de Van, Chausie, Colourpoint Shorthair, Cornish Rex, Cymric, Devon Rex, Don Bald Cat, Don Hairless, Don Sphynx, Donskoy, Egyptian Mau, European Shorthair, Européen, Exotic Shorthair, Exotique à Poil Court, Foldex, Gatto di Ceylon, German Rex, Havana Brown, Highland Fold, Highland Straight, Japanese Bobtail, Javanais, Korat, Kurile Islands Bobtail, Kurilian Bobtail, Laperm, Lynx Domestique, Maine Coon, Malayen, Mandarin, Manx, Mau Egyptien, Munchkin, Nebelung, Neva Masquerade, Norvégien, Norwegian Forest Cat, Ocicat, Ojos Azules, Oriental à Poil Long, Oriental Shorthair, Persan, Peterbald, Petersburg Sphynx, Pixie Bob, Pudelkatze, Ragdoll, Rex Cornish, Rex Devon, Rex Selkirk, Russe, Russian Bobtail, Russian Hairless, Sacré de Birmanie, Safari, Savannah, Scottish à Poil Long, Scottish Fold, Scottish Straight, Selkirk Rex, Serengeti, Seychellois, Si-Sawat, Siamois (ancien type), Siamois (moderne), Siamois Traditionnel, Sibérien, Silver Laces, Singapura, Skogkatt, Snow Shoe, Sokoke, Somali, Spangled Californien, Sphynx, Spotted Mist, Thaï, The Dalles La Perm, Tiffanie, Tiffany Américain, Tiffany Anglais, Tonkinese, Tonkinois, Toyger, Turc Van, Turkish Angora, Turkish Van, York Chocolate.

L'utilisation selon l'invention est plus particulièrement adaptée aux chats de races qui présentent des prédispositions à la maladie polykystique des reins, pathologie souvent liée à une baisse de l'appétit, voire à une anorexie ; il s'agit des chats maine coon, abyssin, siamois, bleu russe, himalayen, et burmese. Cette utilisation est encore particulièrement adaptée à des chats âgés de 7 ans ou plus, et de préférence de 9 ans ou plus, chez qui l'insuffisance rénale est plus fréquente et cause de pertes d'appétit régulières.

L'utilisation selon la présente invention a pour but de traiter de la baisse ou de la perte d'appétit, en particulier, de stimuler l'appétit et/ou traiter l'anorexie et/ou une aversion alimentaire ; et/ou de prévenir et/ou de traiter un état nauséeux et/ou des vomissements ; et/ou de prévenir des désordres induits par une dénutrition chez un animal incluant une perte de poids, une résistance à l'insuline, une intolérance au glucose, une lipidose hépatique...

Chez le chat, la dénutrition se caractérise généralement par un indice corporel inférieur à 3 sur 5 ; une perte de poids supérieure à 5% en un mois ou 10% en 6 mois ; une anorexie de plus de trois jours ; une amyotrophie généralisée et une hypoalbuminémie (L. Yaguiyan-Colliard, Congrès de l'AFVAC 2009 à Lille).

L'utilisation de mirtazapine selon l'invention présente un intérêt marqué pour le traitement de la baisse ou de la perte d'appétit et/ou la dénutrition postopératoires ; en effet, il est fréquent qu'un chat ayant subi une intervention chirurgicale tarde à retrouver une alimentation normale, il est donc avantageux d'y remédier par une injection de mirtazapine administrée avant ou jusqu'à 72h après l'intervention et qui présente une action efficace et prolongée afin d'aider l'animal à accélérer sa convalescence. Bien entendu, si cette injection ne suffit pas, il est possible de la renouveler au moins une fois, 2 à 7 jours après la précédente injection. Le profil pharmacocinétique de la mirtazapine lorsqu'elle est injectée se révèle particulièrement approprié à l'anorexie post-opératoire : à l'issue de la période d'observation clinique qui suit une intervention, le vétérinaire qui constatera que l'animal tarde à s'alimenter pourra lui administrer une injection de mirtazapine. L'effet prolongé observé par ce mode d'administration évite de devoir se rendre trop fréquemment chez le vétérinaire si d'autres administrations s'avèrent nécessaires.

Selon un mode de réalisation particulier de l'invention, elle se rapporte à la mirtazapine pour son utilisation pour le traitement ou la prévention de l'anorexie chez le chat caractérisée en ce que la mirtazapine est formulée dans un médicament injectable représentant un volume administré inférieur ou égal à 3 ml et dont l'injection produit un effet d'au moins 48 heures.

La mirtazapine est formulée dans une composition injectable liquide qui comprend de préférence un véhicule constitué majoritairement par un solvant, c'est-à-dire l'eau, un solvant organique ou le mélange des deux ou une huile végétale, un solvant organique ou le mélange des deux ou encore le mélange de l'eau avec une huile végétale. Dans une variante de l'invention, le véhicule est constitué d'un mélange d'huile végétale et d'un solvant ; le solvant représentant alors de 0 à 30% du mélange solvant/huile végétale.

Parmi les huiles végétales, on peut citer par exemple l'huile de palme, l'huile de maïs, l'huile de coton, l'huile de tournesol, l'huile d'arachide, l'huile d'olive, l'huile de soja, l'huile de carthame, l'huile de coprah, l'huile de sésame, ou parmi les huiles végétales semi-synthétiques obtenues par fractionnement et/ou hydrolyse et/ou estérification totale des huiles végétales naturelles comme par exemple les triglycérides d'acide gras issus d'huiles végétales, comme les triglycérides des acides caprylique, caprique, linoléïque, succinique (vendues sous les dénominations commerciales Miglyol® 810, 812, 818, 820, 829), les esters du propylène glycol et d'acide gras issus d'huile végétale comme les esters du propylène glycol et des acides caprylique et caprique (vendues sous les dénominations commerciales Miglyol® 840), ainsi que leur mélange, ainsi que des esters parmi lesquels la Triacétine (triacétate de glycéryle), l'oléate d'éthyle, par exemple.

Parmi les solvants organiques, on peut citer par exemple l'alcool benzylique, l'éthanol, la N-méthyl pyrrolidone, le glycerol-formal, le glycofurol, le Diethylene glycol monoethyl ether, le propylene glycol, le polyéthylène glycol par exemple, le PEG300, le PEG 200 et le PEG 400.

La sélection du véhicule est réalisée, de manière à former des solutions liquides, en fonction de sa capacité à dissoudre la substance active à température ambiante sans en modifier la structure chimique et la stabilité. Le véhicule choisi doit être biocompatible et adapté à la voie injectable. Le véhicule sera choisi parmi les solvants polaires protiques, les solvants polaires aprotiques, les solvants apolaires aprotiques ou leur mélange.

Selon un mode de réalisation particulier de l'invention, la mirtazapine est en solution dans la composition vétérinaire injectable liquide.

La composition injectable liquide de mirtazapine pourra également comprendre au moins un antioxydant choisi parmi le butylhydroxyanisol (BHA), butylhydroxytoluène (BHT), la vitamine E et ses dérivés, le propylgallate et les mélanges de ceux-ci.

La composition injectable liquide comprend la mirtazapine à une concentration inférieure ou égale à 2% p/v, de préférence comprise entre 0,5 et 1,5% p/v, encore préférentiellement comprise entre 0,75 et 1% p/v ; cette composition injectable liquide de mirtazapine est administrée à un volume qui ne dépasse pas 3 ml, il est de préférence inférieur ou égal à 2 ml et encore préférentiellement compris entre 0,5 et 1 ml.

L'administration de la composition injectable liquide de mirtazapine est telle qu'elle permettra l'administration d'une dose de mirtazapine comprise entre 0,2 et 2 mg/kg, plus particulièrement entre 0,2 et 1,5 mg/kg, ou entre 0,2 et 0,5mg/kg, ou encore entre 0,5 et 1 mg/kg ou encore préférentiellement entre 0,4 et 0,8 mg/kg.

La composition injectable liquide de mirtazapine est de préférence administrée en une dose unique ; alternativement, elle est administrée à plusieurs reprises, de préférence en deux doses, chaque dose étant espacée de 1 à 7 jours ou de 2 à 7 jours, de préférence de 2 à 5 jours, ou de manière plus adaptée les deux injections seront séparées par un intervalle de 24h, 48h ou 72h, bien que la présente invention ne soit pas limitée au schéma d'administration susmentionné.

### Figures

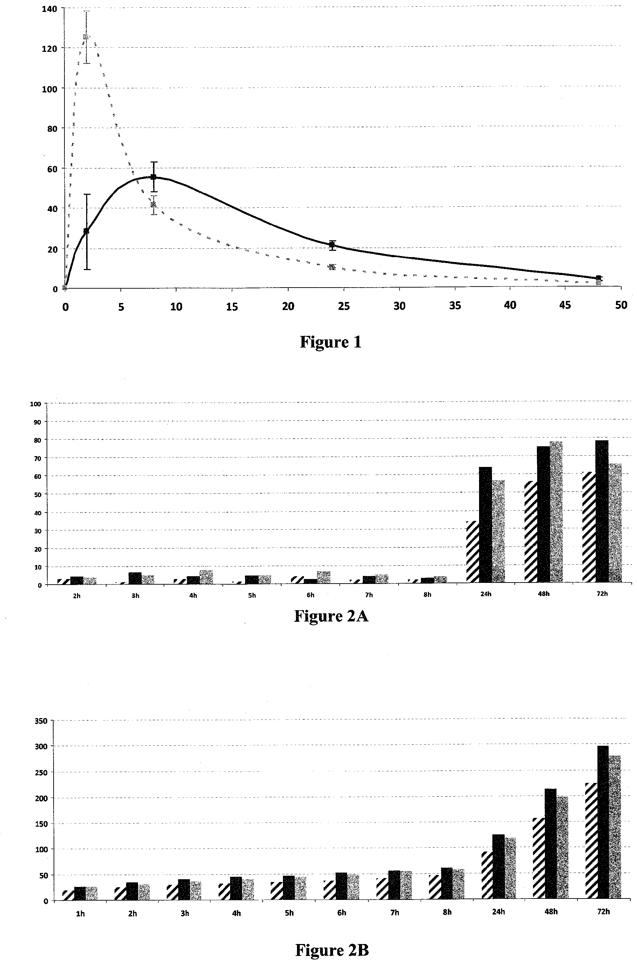

La **Figure 1** est un graphe qui représente la concentration plasmatique en mirtazapine en ng/ml au cours du temps (en heures) chez un chat sain après une administration par injection (courbe noire) et après une administration par voie orale (courbe grise pointillée).
Les **Figures 2A** et **2B** sont des histogrammes représentant la consommation alimentaire en gramme au fil du temps (en heures, au temps de mesure pour 2A et en cumulé pour 2B) des chats après traitement et auxquels on fait subir un stress. Les histogrammes rayés correspondent au groupe contrôle, les histogrammes noirs au groupe ayant reçu le produit par administration injectable selon l'invention, les histogrammes gris au groupe ayant reçu le produit par administration orale selon l'art antérieur.

### Exemple - Essais d'administration de mirtazapine par injection sur chats sains

L'objectif de ces essais est d'évaluer l'effet de l'administration de la mirtazapine par injection sous-cutanée sur l'appétit des chats en comparaison avec une administration par voie orale (contrôle positif) et un témoin (injection de solution saline, contrôle négatif).

Ces essais ont été conduits en respectant les bonnes pratiques de laboratoire établies par le « US Animal Welfare Act » et les directives du « Canadian Council on Animal Care ».

### Matériels et méthodes

### - Produits administrés

La formulation injectable testée est une suspension de mirtazapine à 0,75% poids/volume dans du Miglyol 812. La formulation orale testée est une solution de mirtazapine à 15mg/ml (Summit Veterinary Pharmacy Inc.).

### - protocole

Les chats sains sélectionnés sont préalablement acclimatés pendant au moins sept jours avant les essais ; durant cette période, ils sont examinés par un vétérinaire. Ils sont ensuite répartis en trois groupes de deux chats selon un design expérimental en cross-over, chaque chat recevant chacun des 3 traitements selon une séquence prédéterminée :
- 0,5 ml de solution saline (chlorure de sodium 0,9%) est administré par injection sous-cutanée dans la zone interscapulaire au groupe T0 qui représente le contrôle négatif ;
- le groupe T1 reçoit une injection sous-cutanée dans la zone interscapulaire de 0,5 ml de la formulation testée de mirtazapine (représente une administration de 3,75 mg de mirtazapine) ;
- le groupe T3 reçoit lui 0,25 ml (représente une administration de 3,75 mg de mirtazapine) de la formulation liquide par administration forcée (seringue) dans la gueule et représente le contrôle positif.

Afin de simuler un stress susceptible de provoquer une perte d'appétit des chats, leur environnement a été modifié régulièrement juste avant le démarrage de l'étude. L'évaluation de la consommation des aliments par les chats se fait par pesée de la ration proposée (100 g) à t = 0, 1, 2, 3, 4, 5, 6, 7, 8, 24 et 48h après l'administration ; si la ration est totalement consommée, une nouvelle ration de 100 g est alors proposée.

La détermination de la concentration plasmatique de mirtazapine est faite sur des échantillons de sang prélevés aux jours -1, 0, 14, 28 et 42 et 2, 8, 24 et 48h par rapport à l'administration de mirtazapine.

### Résultats

### - pharmacocinétique

Le graphe de la **Figure 1** représente la concentration plasmatique en mirtazapine après une administration par injection (courbe grise) et après une administration par voie orale (courbe noire).

Il ressort de ce graphe que la pharmacocinétique du produit change sensiblement en fonction du mode d'administration ; l'injection conduit à un Cₘₐₓ plus faible et un Tₘₐₓ plus tardif par rapport à l'administration orale, résultant en un profil plus favorable en terme de durée d'action et de toxicité.

| | **Cmax (µg/L)** | **Tmax (h)** | **AUClast (µg/L*h)** | **Thalf (h)** |
|---|---|---|---|---|
| Formulation injectée | 58.25 | 9.25 | 1054.9 | 10.885 |
| Formulation ingérée | 127.7 | 2 | 1047.7 | 8.8324 |

### - Tolérance locale

L'examen des points d'injection du produit montre une bonne tolérance locale.

### - Consommation alimentaire

La consommation alimentaire est représentée aux **Figures 2A** (consommation alimentaire mesurée à chaque point) et **2B** (consommation alimentaire cumulée).

Ces essais démontrent l'effet prolongé plus important de l'injection comparée à l'administration orale en particulier à partir de 72h après l'administration de la mirtazapine.

Il est donc intéressant de constater que l'administration d'une dose de mirtazapine par injection sous-cutanée est aussi efficace que l'administration de la même dose par voie orale pour induire une augmentation de l'appétit mais que l'injection présente l'avantage d'induire l'appétit pendant plus longtemps, cette efficacité est donc plus durable.

## Revendications

1. Mirtazapine pour son utilisation pour le traitement de la baisse ou de la perte d'appétit et/ou la prévention des désordres induits par une dénutrition chez un chat, **caractérisée en ce que** la mirtazapine est administrée par voie parentérale et formulée dans une composition vétérinaire injectable liquide comprenant une concentration de mirtazapine inférieure ou égale à 2% poids/volume.

2. Mirtazapine pour son utilisation selon l'une quelconque des revendications précédentes, pour stimuler l'appétit.

3. Mirtazapine pour son utilisation selon l'une quelconque des revendications précédentes, pour le traitement de l'anorexie, d'un état nauséeux, de vomissements, et/ou d'une aversion alimentaire.

4. Mirtazapine pour son utilisation selon l'une quelconque des revendications précédentes, pour prévenir la perte de poids.

5. Mirtazapine pour son utilisation selon l'une quelconque des revendications 2 à 4, **caractérisée en ce que** la baisse ou la perte d'appétit et/ou la dénutrition sont postopératoires.

6. Mirtazapine pour son utilisation selon l'une quelconque des revendications précédentes, **caractérisée en ce que** ladite composition vétérinaire injectable liquide comprend véhicule constitué majoritairement par un solvant choisi parmi l'eau, un solvant organique ou le mélange des deux ou une huile végétale, un solvant organique ou le mélange des deux ou encore le mélange de solvant avec une huile végétale, le solvant représentant alors de 0 à 30% du mélange solvant/huile végétale.

7. Mirtazapine pour son utilisation selon l'une quelconque des revendications précédentes, **caractérisée en ce qu'**elle est administrée en une quantité représentant entre 0,2 et 2 mg/kg de poids corporel.

8. Mirtazapine pour son utilisation selon l'une des revendications 1 à 7, **caractérisée en ce qu'**elle est administrée en une quantité représentant entre 0,4 et 0,8 mg/kg de poids corporel.

9. Mirtazapine pour son utilisation selon l'une quelconque des revendications précédentes, **caractérisée en ce qu'**elle est administrée en une dose unique.

10. Mirtazapine pour son utilisation selon l'une quelconque des revendications 1 à 9, **caractérisée en ce qu'**elle est administrée en deux injections séparées par un intervalle de 24h, 48h ou 72h.

11. Mirtazapine pour son utilisation selon l'une quelconque des revendications précédentes, pour le traitement ou la prévention de l'anorexie chez le chat **caractérisée en ce que** la mirtazapine est formulée dans un médicament injectable représentant un volume inférieur ou égale à 3 ml.

## Patentansprüche

1. Mirtazapin für seine Verwendung zur Behandlung von nachlassendem oder Verlust von Appetit und/oder zur Vorbeugung von Störungen, die von einer Mangelernährung bei einer Katze induziert sind, **dadurch gekennzeichnet, dass** das Mirtazapin auf parenteralem Weg verabreicht wird und in einer flüssigen injizierbaren Veterinärzusammensetzung formuliert ist, die eine Konzentration von Mirtazapin von unter oder gleich 2 Gew.-/Vol.-% umfasst.

2. Mirtazapin für seine Verwendung nach einem der vorangehenden Ansprüche zum Anregen des Appetits.

3. Mirtazapin für seine Verwendung nach einem der vorangehenden Ansprüche zur Behandlung der Anorexie, eines Übelkeitszustands, von Erbrechen und/oder einer Nahrungsaversion.

4. Mirtazapin für seine Verwendung nach einem der vorangehenden Ansprüche zum Vorbeugen von Gewichtsverlust.

5. Mirtazapin für seine Verwendung nach einem der Ansprüche 2 bis 4, **dadurch gekennzeichnet, dass** das Nachlassen oder der Verlust von Appetit und/oder die Mangelernährung postoperativ sind.

6. Mirtazapin für seine Verwendung nach einem der vorangehenden Ansprüche, **dadurch gekennzeichnet, dass** die flüssige injizierbare Veterinärzusammensetzung einen Träger umfasst, der mehrheitlich von einem Lösungsmittel gebildet ist, ausgewählt aus Wasser, einen organischen Lösungsmittel oder dem Gemisch beider oder einem Pflanzenöl, einem organischen Lösungsmittel oder dem Gemisch beider oder auch dem Gemisch von Lösungsmittel mit einem Pflanzenöl, wobei das Lösungsmittel dann 0 bis 30 % des Gemischs Lösungsmittel/Pflanzenöl darstellt.

7. Mirtazapin für seine Verwendung nach einem der vorangehenden Ansprüche, **dadurch gekennzeichnet, dass** es in einer Menge verabreicht wird, die zwischen 0,2 und 2 mg/kg Körpergewicht darstellt.

8. Mirtazapin für seine Verwendung nach einem der Ansprüche 1 bis 7, **dadurch gekennzeichnet, dass** es in einer Menge verabreicht wird, die zwischen 0,4 und 0,8 mg/kg Körpergewicht darstellt.

9. Mirtazapin für seine Verwendung nach einem der vorangehenden Ansprüche, **dadurch gekennzeichnet, dass** es in einer Einzeldosis verabreicht wird.

10. Mirtazapin für seine Verwendung nach einem der Ansprüche 1 bis 9, **dadurch gekennzeichnet, dass** es in zwei Injektionen verabreicht wird, die durch ein Intervall von 24, 48 oder 72 Stunden getrennt sind.

11. Mirtazapin für seine Verwendung nach einem der vorangehenden Ansprüche zur Behandlung oder zur Vorbeugung der Anorexie bei der Katze, **dadurch gekennzeichnet, dass** das Mirtazapin in einem injizierbaren Arzneimittel formuliert ist, das ein Volumen von unter oder gleich 3 ml aufweist.

## Claims

1. Mirtazapine for use for the treatment of the reduction or loss of appetite and/or the prevention of disorders induced by malnutrition in a cat, **characterised in that** mirtazapine is administered parenterally and formulated in a liquid injectable veterinary composition comprising a concentration of mirtazapine of less than or equal to 2% weight/volume.

2. Mirtazapine for use according to any one of the preceding claims, to stimulate the appetite.

3. Mirtazapine for use according to any one of the preceding claims, for the treatment anorexia, nausea, vomiting, and/or food aversion.

4. Mirtazapine for use according to any one of the preceding claims, to prevent weight loss.

5. Mirtazapine for use according to any one of claims 2 to 4, **characterised in that** the reduction or loss of appetite and/or malnutrition are postoperative.

6. Mirtazapine for use according to any one of the preceding claims, **characterised in that** said liquid injectable veterinary composition comprises vehicle mainly made up of a solvent selected from water, an organic solvent or the mixture of the two or a vegetable oil, an organic solvent or the mixture of the two or even the mixture of the solvent with a vegetable oil, the solvent then representing 0 to 30% of the solvent/vegetable oil mixture.

7. Mirtazapine for use according to any one of the preceding claims, **characterised in that** it is administered in a quantity representing between 0.2 and 2mg/kg of body weight.

8. Mirtazapine for use according to one of claims 1 to 7, **characterised in that** it is administered in a quantity representing between 0.4 and 0.8mg/kg of body weight.

9. Mirtazapine for use according to any one of the preceding claims, **characterised in that** it is administered in a single dose.

10. Mirtazapine for use according to any one of claims 1 to 9, **characterised in that** it is administered in two injections separated by an interval of 24 hours, 48 hours or 72 hours.

11. Mirtazapine for use according to any one of the preceding claims, for the treatment or prevention of anorexia in a cat **characterised in that** mirtazapine is formulated in an injectable drug representing a volume of less than or equal to 3ml.
